# EUROPEAN PATENT APPLICATION

(11) **EP 2 657 347 A1**
(43) Date of publication of application: **30.10.2013**
(21) Application number: 12165696.1
(22) Date of filing: 26.04.2012
(51) Int. Cl.: C12Q 1/68

(54) **Electrochemical competitive assay and use thereof**

(71) Applicant: Université Paris Diderot - Paris 7, 75205 Paris Cedex 13 (FR)
(72) Inventor: Challier, Lylian, 75014 Paris (FR); Fave, Claire, 75019 Paris (FR); Limoges, Benoît, 91220 Brétigny-sur-Orge (FR); Marchal, Damien, 75009 Paris (FR); Mavré, François, 93500 Pantin (FR); Moreau, Julie, 75020 Paris (FR); Noël, Vincent, 95300 Pontoise (FR); Schöllhorn, Bernd, 91430 Igny (FR)
(74) Representative: Icosa

(57) **Abstract**

The present invention relates to an electrochemical competitive assay, kits, methods and use thereof.

SEQUENCE LISTING

<110> UNIVERSITE PARIS DIDEROT - PARIS 7

<120> ELECTROCHEMICAL COMPETITIVE ASSAY AND USE THEREOF

<130> CV - 182/EP

<160> 48

<170> BiSSAP 1.0

<210> 1
<211> 49
<212> DNA
<213> artificial sequences

<220>
<221> source
<222> 1..49
<223> /mol_type="DNA"
      /note="Apt49-T"
      /organism="artificial sequences"

<400> 1
aattcgctag ctggagcttg gattgatgtg gtgtgtgagt gcggtgccc                49

<210> 2
<211> 49
<212> DNA
<213> artificial sequences

<220>
<221> source
<222> 1..49
<223> /mol_type="DNA"
      /note="Scramble"
      /organism="artificial sequences"

<400> 2
ctatgaccct acctgctgat gcgtagcgag acccggaatc taacccccct                49

<210> 3
<211> 38
<212> DNA
<213> artificial sequences

<220>
<221> source
<222> 1..38
<223> /mol_type="DNA"
      /note="aptamer"
      /organism="artificial sequences"

<400> 3
acagctgggt gaagtaactt cctaaaagga acagaggg                38

<210> 4
<211> 68
<212> RNA
<213> artificial sequences

<220>
<221> source
<222> 1..68
<223> /mol_type="RNA"
      /note="aptamer"
      /organism="artificial sequences"

<400> 4
ggcaccaaag cugaaguagc ggauaacuca aauuacuuua gguguaugaa ggugaaacua      60

gcaaugaa                                                                68


<210> 5
<211> 71
<212> DNA
<213> artificial sequences

<220>
<221> source
<222> 1..71
<223> /mol_type="DNA"
      /note="aptamer"
      /organism="artificial sequences"

<400> 5
ggaattcgct agcacgttga cgctggtgcc cggttgtggt gcgagtgttg tgtggatccg      60

agctccacgt g                                                            71


<210> 6
<211> 36
<212> DNA
<213> artificial sequences

<220>
<221> source
<222> 1..36
<223> /mol_type="DNA"
      /note="aptamer"
      /organism="artificial sequences"

<400> 6
gatcgggtgt gggtggcgta aagggagcat cggaca                                 36


<210> 7
<211> 60
<212> DNA
<213> artificial sequences

<220>
<221> source
<222> 1..60
<223> /mol_type="DNA"
      /note="aptamer"
      /organism="artificial sequences"

<400> 7
aatcgcatta ccttatacca gcttattcaa ttacgtctgc acataccagc ttattcaatt      60


<210> 8
<211> 63

<212> DNA
<213> artificial sequences

<220>
<221> source
<222> 1..63
<223> /mol_type="DNA"
      /note="aptamer"
      /organism="artificial sequences"

<400> 8
ccggtgggtg gtcaggtggg atagcgttcc gcgtatggcc cagcgcatca cgggttcgca      60

cca                                                                    63


<210> 9
<211> 39
<212> DNA
<213> artificial sequences

<220>
<221> source
<222> 1..39
<223> /mol_type="DNA"
      /note="aptamer"
      /organism="artificial sequences"

<400> 9
atgaggcggg gtaaaattgc tccgataaaa acgcaaagt                             39


<210> 10
<211> 59
<212> DNA
<213> artificial sequences

<220>
<221> source
<222> 1..59
<223> /mol_type="DNA"
      /note="aptamer"
      /organism="artificial sequences"

<400> 10
gagagagggg agacaagcgt gtaggatggg gagggcgggt ggctgcgatt gtttcccca       59


<210> 11
<211> 59
<212> DNA
<213> artificial sequences

<220>
<221> source
<222> 1..59
<223> /mol_type="DNA"
      /note="aptamer"
      /organism="artificial sequences"

<400> 11
actggggatg ggtggggagg gctgggatgc aaattcggca gtggaatcgt gtcagactc       59


<210> 12

<211> 100
<212> DNA
<213> artificial sequences

<220>
<221> source
<222> 1..100
<223> /mol_type="DNA"
      /note="aptamer"
      /organism="artificial sequences"

<400> 12
gtaccagctt attcaattac agatcgaggg cagcgatagc tgggctaata aggttagccc      60

catcggtcct ggacttggga ctagatagta tgttcatcag                            100


<210> 13
<211> 99
<212> DNA
<213> artificial sequences

<220>
<221> source
<222> 1..99
<223> /mol_type="DNA"
      /note="aptamer"
      /organism="artificial sequences"

<400> 13
gtaccagctt attcaattag cgccgattga cccaaatcgt tttgtatgca aaagcgctgc      60

tggtatcaac tgttacccat gaagatagta gttcatcag                             99


<210> 14
<211> 60
<212> DNA
<213> artificial sequences

<220>
<221> source
<222> 1..60
<223> /mol_type="DNA"
      /note="aptamer"
      /organism="artificial sequences"

<400> 14
aaaagggaag aaactgcgcg ctcagctggt ttagctgggg gcggcttttg agaaggcgtt      60


<210> 15
<211> 60
<212> DNA
<213> artificial sequences

<220>
<221> source
<222> 1..60
<223> /mol_type="DNA"
      /note="aptamer"
      /organism="artificial sequences"

<400> 15
aaaagggaag aaactgcgcg ctccgctggt ttagctgggg gcggcttttg agaaggtgtt      60

<210> 16
<211> 70
<212> RNA
<213> artificial sequences

<220>
<221> source
<222> 1..70
<223> /mol_type="RNA"
      /note="aptamer"
      /organism="artificial sequences"

<400> 16
agacacugug acagcuggcc cacaagccuc gcgccccgac uuguagcucc aaucuuuccc      60

ccguacaagc                                                              70


<210> 17
<211> 69
<212> RNA
<213> artificial sequences

<220>
<221> source
<222> 1..69
<223> /mol_type="RNA"
      /note="aptamer"
      /organism="artificial sequences"

<400> 17
gccuacaacu agccuuccgu aggcgccagg cuggcuuucg aaacauaacu agacaaguca      60

uagcgacca                                                               69


<210> 18
<211> 71
<212> DNA
<213> artificial sequences

<220>
<221> source
<222> 1..71
<223> /mol_type="DNA"
      /note="aptamer"
      /organism="artificial sequences"

<400> 18
gccagcggac aaatccggtg cgcataacca cctcagtgcg agcaacgatg gcctttctac      60

ccaaagattt t                                                            71


<210> 19
<211> 72
<212> DNA
<213> artificial sequences

<220>
<221> source
<222> 1..72
<223> /mol_type="DNA"

/note="aptamer"
      /organism="artificial sequences"

<400> 19
ccagcggaac gaaacagggt gtccagcttg tggacgagac aggttcatgg gagctattcg      60

catcgcaaaa tc                                                          72


<210> 20
<211> 35
<212> RNA
<213> artificial sequences

<220>
<221> source
<222> 1..35
<223> /mol_type="RNA"
      /note="aptamer"
      /organism="artificial sequences"

<400> 20
ggcguguagg auaucguguu cgagaaggac acgcc                                 35


<210> 21
<211> 81
<212> DNA
<213> artificial sequences

<220>
<221> source
<222> 1..81
<223> /mol_type="DNA"
      /note="aptamer"
      /organism="artificial sequences"

<400> 21
cgcatttaca agaaatatgt tcatcgcgtt cgtcgcagtc cgaagaactg atatcatcca      60

caaagcgtcg ggtgctaagc g                                                81


<210> 22
<211> 79
<212> DNA
<213> artificial sequences

<220>
<221> source
<222> 1..79
<223> /mol_type="DNA"
      /note="aptamer"
      /organism="artificial sequences"

<400> 22
caagtttaca agaaagatgc accaacgcgg ctaacgggag ctcaaagctg agaactaccg      60

acaacaaagc tcatggcaa                                                   79


<210> 23
<211> 44
<212> RNA
<213> artificial sequences

<220>
<221> source
<222> 1..44
<223> /mol_type="RNA"
      /note="aptamer"
      /organism="artificial sequences"

<400> 23
cgaucggcga cucacucucc agugauucgg guagaaacug cgaa                        44


<210> 24
<211> 39
<212> RNA
<213> artificial sequences

<220>
<221> source
<222> 1..39
<223> /mol_type="RNA"
      /note="aptamer"
      /organism="artificial sequences"

<400> 24
cgccagaacg gaccauggug guagaaacug cguccugug                              39


<210> 25
<211> 43
<212> RNA
<213> artificial sequences

<220>
<221> source
<222> 1..43
<223> /mol_type="RNA"
      /note="aptamer"
      /organism="artificial sequences"

<400> 25
uuccucugau acguggaaga aacugcgaac uccuaguucg gag                         43


<210> 26
<211> 61
<212> RNA
<213> artificial sequences

<220>
<221> source
<222> 1..61
<223> /mol_type="RNA"
      /note="aptamer"
      /organism="artificial sequences"

<400> 26
aauccgguug ccgcugauua caucaugugg agagcuugau acuauugagg augcuuguga      60

g                                                                       61


<210> 27
<211> 65
<212> RNA

<213> artificial sequences

<220>
<221> source
<222> 1..65
<223> /mol_type="RNA"
      /note="aptamer"
      /organism="artificial sequences"

<400> 27
gggagaauuc ccgcggcugg ugcgugcagg acgucgaucg aauccgccug cagugcacgg     60

cuccc                                                                 65


<210> 28
<211> 65
<212> RNA
<213> artificial sequences

<220>
<221> source
<222> 1..65
<223> /mol_type="RNA"
      /note="aptamer"
      /organism="artificial sequences"

<400> 28
gggagaauuc ccgcggcaug agucaccgcc gucgagcuuc cggcgugcug cagugcacgg     60

cuccc                                                                 65


<210> 29
<211> 16
<212> RNA
<213> artificial sequences

<220>
<221> source
<222> 1..16
<223> /mol_type="RNA"
      /note="aptamer"
      /organism="artificial sequences"

<400> 29
ucucugccac acguac                                                     16


<210> 30
<211> 16
<212> RNA
<213> artificial sequences

<220>
<221> source
<222> 1..16
<223> /mol_type="RNA"
      /note="aptamer"
      /organism="artificial sequences"

<220>
<221> misc_feature
<222> 6
<223> /note="n is A or G or C or U"

<400> 30
cucucngccc acguac                                                       16


<210> 31
<211> 114
<212> DNA
<213> artificial sequences

<220>
<221> source
<222> 1..114
<223> /mol_type="DNA"
      /note="aptamer"
      /organism="artificial sequences"

<400> 31
ttggcgttgg catgacgcgg ggaatcgggt gcatcgatga ctactcctgg gcccacgtct      60

gttgttgacg tcacagcttg atttaggata gcgcttgggc agtcgtgcag tgga           114


<210> 32
<211> 66
<212> DNA
<213> artificial sequences

<220>
<221> source
<222> 1..66
<223> /mol_type="DNA"
      /note="aptamer"
      /organism="artificial sequences"

<400> 32
aggcggggtg acgtacggaa ttcgctagct ggagcttgga ttgatgtggt gtgtgagtgc      60

ggtccc                                                                  66


<210> 33
<211> 72
<212> DNA
<213> artificial sequences

<220>
<221> source
<222> 1..72
<223> /mol_type="DNA"
      /note="aptamer"
      /organism="artificial sequences"

<400> 33
cccaacgagc ggcgtacgga attaattcgc tagctggagc ttggattgat gtggtgtgtc      60

agtgcggagc cc                                                           72


<210> 34
<211> 80
<212> DNA
<213> artificial sequences

<220>

<221> source
<222> 1..80
<223> /mol_type="DNA"
      /note="aptamer"
      /organism="artificial sequences"

<400> 34
ttaacctaat ggaatacaat tctaacagga ataggagggt agctccaagt actaacgacc     60

gttccggtag ccggctggct                                                 80


<210> 35
<211> 73
<212> DNA
<213> artificial sequences

<220>
<221> source
<222> 1..73
<223> /mol_type="DNA"
      /note="aptamer"
      /organism="artificial sequences"

<400> 35
cgaattgccg ttgcaccgca tgtcaaatca tgcggcggtt acatgtcgac ggctcggaaa     60

aatcggacag cgc                                                        73


<210> 36
<211> 30
<212> DNA
<213> artificial sequences

<220>
<221> source
<222> 1..30
<223> /mol_type="DNA"
      /note="aptamer"
      /organism="artificial sequences"

<400> 36
accgcggggt tgcggaccgg gagctccagc                                      30


<210> 37
<211> 30
<212> DNA
<213> artificial sequences

<220>
<221> source
<222> 1..30
<223> /mol_type="DNA"
      /note="aptamer"
      /organism="artificial sequences"

<400> 37
gaattgccgt aatttcccgt ggagcgatgc                                      30


<210> 38
<211> 74
<212> RNA

<213> artificial sequences

<220>
<221> source
<222> 1..74
<223> /mol_type="RNA"
      /note="aptamer"
      /organism="artificial sequences"

<400> 38
uguaacucua ggcucccgug gaucgcauuu ggacuucugc cuaacgcgug ugcuagagag      60

caggcaaaca agug                                                        74


<210> 39
<211> 74
<212> RNA
<213> artificial sequences

<220>
<221> source
<222> 1..74
<223> /mol_type="RNA"
      /note="aptamer"
      /organism="artificial sequences"

<400> 39
accccuuugu gggaacgcaa aacuaaagga agaucucaag aaagguggau cgcauuugga      60

cuucugccca gggu                                                        74


<210> 40
<211> 91
<212> RNA
<213> artificial sequences

<220>
<221> source
<222> 1..91
<223> /mol_type="RNA"
      /note="aptamer"
      /organism="artificial sequences"

<400> 40
gggagaggau acuacacgug auacucgucc guaggaugac cagcacuaac uagucacaag      60

ucggguccau ugcauguagc agaagcuugg c                                     91


<210> 41
<211> 60
<212> RNA
<213> artificial sequences

<220>
<221> source
<222> 1..60
<223> /mol_type="RNA"
      /note="aptamer"
      /organism="artificial sequences"

<400> 41
cugguuaguu uugcacagug gucgaugcua gacuugguuu agguaaugag uccaauaguc      60

<210> 42
<211> 60
<212> RNA
<213> artificial sequences

<220>
<221> source
<222> 1..60
<223> /mol_type="RNA"
      /note="aptamer"
      /organism="artificial sequences"

<400> 42
aguauagcga gguuuagcua cacucgugcu gaucguuugg uacgggaccu gcguguagcc     60


<210> 43
<211> 30
<212> DNA
<213> artificial sequences

<220>
<221> source
<222> 1..30
<223> /mol_type="DNA"
      /note="aptamer"
      /organism="artificial sequences"

<400> 43
accgcggggt tgcggaccgg gagctccagc                                      30


<210> 44
<211> 30
<212> DNA
<213> artificial sequences

<220>
<221> source
<222> 1..30
<223> /mol_type="DNA"
      /note="aptamer"
      /organism="artificial sequences"

<400> 44
gaattgccgt aatttcccgt ggagcgatgc                                      30


<210> 45
<211> 40
<212> DNA
<213> artificial sequences

<220>
<221> source
<222> 1..40
<223> /mol_type="DNA"
      /note="aptamer"
      /organism="artificial sequences"

<400> 45
tgatgtctcg aaatgaagga ggaggagggg aaccggatgg                           40

<210> 46
<211> 40
<212> DNA
<213> artificial sequences

<220>
<221> source
<222> 1..40
<223> /mol_type="DNA"
      /note="aptamer"
      /organism="artificial sequences"

<400> 46
agatgtctga agaactcgga ggaggaggcg gtctggcctg                              40


<210> 47
<211> 65
<212> RNA
<213> artificial sequences

<220>
<221> source
<222> 1..65
<223> /mol_type="RNA"
      /note="aptamer"
      /organism="artificial sequences"

<400> 47
gcuuaaagcc uaaaacauac cagaucgcca cccgcgcuuu aaucuggaga ggugaagaau      60

ucgac                                                                  65


<210> 48
<211> 63
<212> RNA
<213> artificial sequences

<220>
<221> source
<222> 1..63
<223> /mol_type="RNA"
      /note="aptamer"
      /organism="artificial sequences"

<400> 48
uuaaagccua aaacauacca gaucgcuacc cgcgcuuuaa ucuggggagg ugaagaauuc      60

gac                                                                    63

## Description

### FIELD OF INVENTION

The present invention relates to an electrochemically competitive assay and use thereof for detecting and/or quantifying the presence of an analyte or a target in a sample.

### BACKGROUND OF INVENTION

Detection and quantification methods of an analyte or a target in a sample are well known in the art, as for example enzyme-linked immunoassay (ELISA). Competitive assays based on these methods were also developed, wherein the formation of a complex involving a labeled reagent is inhibited in the presence of an analyte in an analyte-concentration dependent manner or wherein a pre-existing complex involving a labeled reagent is disrupted in the presence of an analyte in an analyte-concentration dependent manner. These methods are usually based on indirect monitoring of the enzyme label via spectrophotometry, fluorimetry or radiometry.

To directly monitor the activity of the enzyme labeled and thus improve the sensitivity of detection, methods based on electrochemical measurement were developed, such as in US5,149,630.

In order to develop faster and automated assays, WO2009/015291 described another method for electrochemical detection of an analyte in a sample, which is based on the discovery that asymmetric distribution of a redox enzyme between two electrodes that occurs when a redox enzyme containing reagent is immobilized at the surface of one electrode can be detected as a chemical potential gradient arising from an asymmetry in the distribution of oxidized or reduced redox substrate.

However, there is a need to develop other methods that are not based on enzyme labeling, to improve significantly sensitivity of the detection.

WO2011/147563 thus provides a homogeneous immunoassay using two different conjugates as reagents: one conjugate comprising a redox marker and an analyte molecule and the other conjugate comprising an anti-redox marker antibody and an antibody that specifically binds the analyte. In the presence of the analyte in the sample, the redox activity of the redox active conjugate is reduced after binding to the anti-redox marker antibody.

The present invention hereby provides an alternative assay for detecting and/or quantifying the presence of an analyte or a target in a sample, said assay being rapid, simple and highly sensitive.

### SUMMARY

One object of the present invention is an electrochemical competitive assay for assessing and/or quantifying the presence of at least one target in a sample, comprising
- at least one oligonucleotide ligand capable of specifically interacting with the target of interest,
- at least one redox-labeled target or redox-labeled target analog,
wherein the oligonucleotide ligand and the redox-labeled target or redox-labeled target analog form a first complex allowing a first redox signal to be electrochemically detectable and
wherein, when the sample to be tested is added, the first complex is disrupted by the target present in the sample allowing a second redox signal to be electrochemically detectable,
said second redox signal allowing the assessment and/or quantification of the target presence in the sample.

In one embodiment, said electrochemical competitive assay is an homogeneous assay. In one embodiment, said electrochemical competitive assay is an heterogeneous assay, wherein the oligonucleotide ligand is attached to a solid phase or to an electrode.

In another embodiment, said oligonucleotide ligand is an aptamer, a DNAzyme, a ribozyme, a PNA, or a LNA.

In another embodiment, said redox label is an organic redox label, an organo-metallic redox label or a biological label.

In one embodiment, said sample is a biological sample, a sample derived from an environmental source or a sample containing a chemical compound.

Another object of the present invention is a method for assessing/quantifying the presence of at least one target in a sample comprising the steps of:
- contacting the oligonucleotide ligand with the redox labeled target or redox-labeled target analog in a detector comprising an electrode structure,
- detecting electrochemically a first redox signal, wherein the first redox signal is indicative of the complexes formed by the oligonucleotide ligand and the redox labeled target or redox-labeled target analog,
- contacting the sample with the detector,
- detecting electrochemically a second redox signal, wherein the second redox signal is indicative of the disruption of the previously formed complexes and thus of the presence of the target in the sample.

In one embodiment, the method further comprises the step of running the assay with at least two standards samples, thereby obtaining a calibration curve.

In one embodiment, the method is for multiplex detection and/or quantification of multiple molecular targets in a sample, wherein the redox label used are different and have a redox potential differing by at least 50 millivolts.

Another object of the invention is a kit of parts comprising:
- a detector comprising an electrode structure,
- at least one oligonucleotide ligand,
- at least one redox labeled target or redox labeled target analog.

In one embodiment of the invention, the oligonucleotide ligand and the redox labeled target or redox labeled target analog are in two separate containers.

In another embodiment of the invention, the oligonucleotide ligand and the redox labeled target or redox labeled target analog are lyophilized and contained in the detector.

In another embodiment, the oligonucleotide ligand is attached to a solid phase or to an electrode and the redox labeled target or redox labeled target analog is in a separate container.

In another embodiment, said kit is for implementing a multiplex method, wherein the detector comprises multiple electrode structures to which are attached multiple oligonucleotide ligands and wherein the multiple redox labeled targets or redox labeled target analogs are in separate containers, the multiple redox label used being different and having a redox potential differing by at least 50 millivolts.

Another object of the invention is the use of the assay, methods and kits as described here above for assessing and/or quantifiying enantiomers or an enantiomeric excess in a sample.

### DETAILED DESCRIPTION

The present invention relates to an electrochemical competitive assay for assessing and/or quantifying the presence of at least one target in a sample, comprising
- at least one oligonucleotide ligand capable of specifically interacting with the target of interest,
- at least one redox-labeled target or redox-labeled target analog.

In the absence of the target, the oligonucleotide ligand and the redox-labeled target or redox-labeled target analog form a first complex and a first redox signal can be electrochemically detected.

In the presence of the target, this first complex is disrupted by the target present in the sample and a second redox signal can be electrochemically detected.

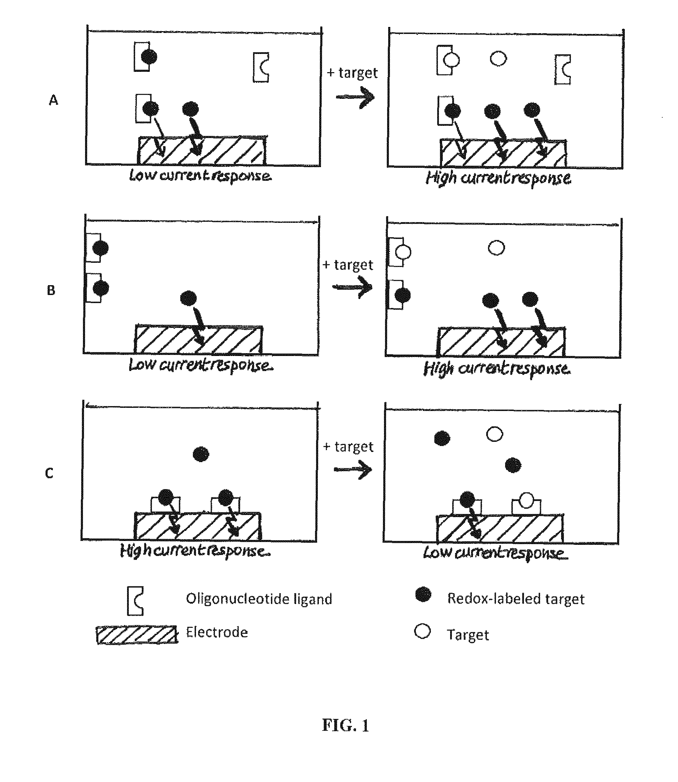

The comparison between said first and said second redox signal allows the assessment and/or quantification of the target presence in the sample (**Figure 1**).

In one embodiment of the invention, the electrochemically competitive assay of the invention is in a homogeneous format (**Figure 1A**). The presence of the target disrupts the complex formed between the oligonucleotide ligand and the redox-labeled target, thereby inducing a better electrochemical detection of the redox label. Thus, in the presence of the target, the second redox signal is increased compared to the first redox signal.

In another embodiment of the invention, the electrochemically competitive assay is in a heterogeneous format, wherein the oligonucleotide ligand is attached to a solid phase (**Figure 1B**). Examples of solid phase include, but are not limited to, wells, membranes, beads.... The presence of the target disrupts the complex formed between the oligonucleotide ligand and the redox-labeled target, thereby inducing a better electrochemical detection of the redox label. Thus, in the presence of the target, the second redox signal is increased compared to the first redox signal.

In another embodiment of the invention, the electrochemically competitive assay is in a heterogeneous format, wherein the oligonucleotide ligand is attached to an electrode, preferably to the working electrode (**Figure 1C**).

Immobilization of the oligonucleotide ligand may be carried out directly or indirectly via a spacer. It is generally achieved by conventional techniques such as for example adsorption, covalent bonding or cross-linking, or combination thereof. It is however essential that immobilization of the oligonucleotide ligand does not adversely affect or prevent the complex formation between the oligonucleotide ligand and the target or redox-labeled target.

The presence of the target disrupts the complex formed between the oligonucleotide ligand and the redox-labeled target, thereby reducing the electrochemical detection of the redox label. Thus, in the presence of the molecular target, the second redox signal is reduced compared to the first redox signal.

As used herein, the term "target" refers to any molecular target such as ion, small organic molecule, oligonucleotide, protein or amino acids, or a cell, virus or any other target that binds to a specific oligonucleotide of the invention. Examples of targets include, but are not limited to, proteins, peptides, glycoproteins, hormones, receptors, antibodies, growth factors, nucleic acids, nucleotides or derivatives thereof, oligosaccharides, carbohydrates, cofactors, viruses, nutrients, metabolites, antibiotics, drugs, toxins (such as, for example, mycotoxins), pesticides, biohazards...

Examples of small organic molecules are ethanolamine, theophylline, malachite green, sulforhodamine B, hematoporphyrin, cholic acid, 4,40-methylenedianiline, dopamine, cocaine, aspartame, thalidomide, (R)-thalidomide, 17(β)-estradiol. Examples of nucleotides are adenine, ATP, xanthine, cAMP. Examples of cofactors are coenzyme A, cyanocobalamin, riboflavin, FMN, FAD, NAD, s-adenosyl methionin, s-adenosyl homocystein, biotin, hemin. Examples of amino acids are L-arginine, L-citrulline, L-valine, L-isoleucine, D-tryptophan, L-tyrosinamide, L-histidine, L-dopa. Examples of carbohydrates are cellobiose, sialyl lewis X, chitin, sialyllactose, sephadex. Examples of antibiotics are kanamycin A, kanamycin B, streptomycin, neomycin, tobramycin, lividomycin, moenomycin A, tetracycline, chloramphenicol, oxytetracycline. Examples of mycotoxins are ochratoxin A, fumonisin B 1. Other examples of targets are bisphenol A, melamine, atrazine.

As used herein, the term "target analog" refers to a species capable of complexing with the same oligonucleotide ligand as the target under assay.

As used herein, the term "oligonucleotide" refers to any polymer of nucleotides, nucleosides or nucleobases that is capable of forming double-stranded complexes through hydrogen bonds between nucleobases. Oligonucleotides include, but are not limited to, DNA, RNA, aptamers, ribozymes, DNAzymes, modified or synthetic DNA or RNA (including but not limited to nucleic acids comprising synthetic and naturally-occurring base analogs, or other sugars, and thiols). Other exemplary oligonucleotides include degradation-resistant polynucleoside amides, peptide nucleic acids (PNAs), locked nucleic acids (LNAs) and other nucleobase-containing polymers. A common PNA has a backbone consisting of repeating N-(2-aminoethyl) glycine unit, linked by amide bonds. Unlike DNA or RNA, PNA often does not contain any sugar or phosphate groups.

The term "oligonucleotide" in no way defines or limits the length of the nucleic acids that may be used to practice the invention.

In an embodiment of the invention, the oligonucleotide ligand is an aptamer, which term describes a specific subset of oligonucleotides selected or designed for specific binding purposes. In one embodiment, aptamers have a length from 5 to 200 nucleic acids, preferably from 5 to 100 nucleic acids, more preferably from 5 to 50 nucleic acids, or more preferably from 5 to 25 nucleic acids.

DNA or RNA aptamers are readily available and can be found for example in the aptamer database (http://aptamer.icmb.utexas.edu/). In vitro selection techniques can also be used to isolate highly affinitive RNA or DNA aptamers that bind almost any arbitrary small molecule, biomacromolecule or cell type. In an embodiment, the aptamer may be rationally dissected into two halves, with one immobilized at the surface of a solid phase or at the surface of an electrode.

Examples of aptamers that can be used in the present invention are the following:

| **Target** | **aptamer** | **SEQ ID NO** |
|---|---|---|
| Cocaine | ACAGCTGGGTGAAGTAACTTCCTAAAA GGAACAGAGGG | SEQ ID NO: 3 |
| Chloramphenicol | GGCACCAAAGCUGAAGUAGCGGAUAA CUCAAAUUACUUUAGGUGUAUGAAG GUGAAACUAGCAAUGAA | SEQ ID NO: 4 |
| Oxytetracycline | GGAATTCGCTAGCACGTTGACGCTGGT GCCCGGTTGTGGTGCGAGTGTTGTGTG GATCCGAGCTCCACGTG | SEQ ID NO: 5 |
| Ochratoxin A | GATCGGGTGTGGGTGGCGTAAAGGGA GCATCGGACA | SEQ ID NO: 6 |
| Fumonisin B1 | AATCGCATTACCTTATACCAGCTTATT CAATTACGTCTGCACATACCAGCTTAT TCAATT | SEQ ID NO: 7 |
| Bisphenol A | CCGGTGGGTGGTCAGGTGGATAGCG TTCCGCGTATGGCCCAGCGCATCACGG GTTCGCACCA | SEQ ID NO: 8 |
| Atrazine | ATGAGGCGGGGTAAAATTGCTCCGAT AAAAACGCAAAGT | SEQ ID NO: 9 |
| Hematoporphyrin | GAGAGAGGGGAGACAAGCGTGTAGGA TGGGGAGGGCGGGTGGCTGCGATTGTT TCCCCA | SEQ ID NO: 10 |
| | ACTGGGGATGGGTGGGGAGGGCTGGG ATGCAAATTCGGCAGTGGAATCGTGTC AGACTC | SEQ ID NO: 11 |
| Cholic acid | GTACCAGCTTATTCAATTACAGATCGA GGGCAGCGATAGCTGGGCTAATAAGG TTAGCCCCATCGGTCCTGGACTTGGGA CTAGATAGTATGTTCATCAG | SEQ ID NO: 12 |
| | GTACCAGCTTATTCAATTAGCGCCGAT TGACCCAAATCGTTTTGTATGCAAAAG CGCTGCTGGTATCAACTGTTACCCATG AAGATAGTAGTTCATCAG | SEQ ID NO: 13 |
| ATP (adenosine) | AAAAGGGAAGAAACTGCGCGCTCAGC TGGTTTAGCTGGGGGCGGCTTTTGAGA AGGCGTT | SEQ ID NO: 14 |
| | AAAAGGGAAGAAACTGCGCGCTCCGC TGGTTTAGCTGGGGGCGGCTTTTGAGA AGGTGTT | SEQ ID NO: 15 |
| Coenzyme A | AGACACUGUGACAGCUGGCCCACAAG CCUCGCGCCCCGACUUGUAGCUCCAA UCUUUCCCCCGUACAAGC | SEQ ID NO: 16 |
| | GCCUACAACUAGCCUUCCGUAGGCGC CAGGCUGGCUUUCGAAACAUAACUAG ACAAGUCAUAGCGACCA | SEQ ID NO: 17 |
| Cyanocobalamin | GCCAGCGGACAAATCCGGTGCGCATA ACCACCTCAGTGCGAGCAACGATGGC CTTTCTACCCAAAGATTTT | SEQ ID NO: 18 |
| | CCAGCGGAACGAAACAGGGTGTCCAG CTTGTGGACGAGACAGGTTCATGGGA GCTATTCGCATCGCAAAATC | SEQ ID NO: 19 |
| FMN | GGCGUGUAGGAUAUCGUGUUCGAGA AGGACACGCC | SEQ ID NO: 20 |
| FAD | CGCATTTACAAGAAATATGTTCATCGC GTTCGTCGCAGTCCGAAGAACTGATAT CATCCACAAAGCGTCGGGTGCTAAGC G | SEQ ID NO: 21 |
| | CAAGTTTACAAGAAAGATGCACCAAC GCGGCTAACGGAGCTCAAAGCTGAG AACTACCGACAACAAAGCTCATGGCA A | SEQ ID NO: 22 |
| S-adenosyl methionine | CGAUCGGCGACUCACUCUCCAGUGAU UCGGGUAGAAACUGCGAA | SEQ ID NO: 23 |
| | CGCCAGAACGGACCAUGGUGGUAGAA ACUGCGUCCUGUG | SEQ ID NO: 24 |
| S-adenosyl homocystein | UUCCUCUGAUACGUGGAAGAAACUGC GAACUCCUAGUUCGGAG | SEQ ID NO: 25 |
| | AAUCCGGUUGCCGCUGAUUACAUCAU GUGGAGAGCUUGAUACUAUUGAGGA UGCUUGUGAG | SEQ ID NO: 26 |
| L-arginine | GGGAGAAUUCCCGCGGCUGGUGCGUG CAGGACGUCGAUCGAAUCCGCCUGCA GUGCACGGCUCCC | SEQ ID NO: 27 |
| | GGGAGAAUUCCCGCGGCAUGAGUCAC CGCCGUCGAGCUUCCGGCGUGCUGCA GUGCACGGCUCCC | SEQ ID NO: 28 |
| L-isoleucine | UCUCUGCCACACGUAC | SEQ ID NO: 29 |
| | CUCUCNGCCCACGUAC | SEQ ID NO: 30 |
| D-Tryptophan | TTGGCGTTGGCATGACGCGGGAATCG GGTGCATCGATGACTACTCCTGGGCCC ACGTCTGTTGTTGACGTCACAGCTTGA TTTAGGATAGCGCTTGGGCAGTCGTGC AGTGGA | SEQ ID NO: 31 |
| L-tyrosinamide | AGGCGGGGTGACGTACGGAATTCGCT AGCTGGAGCTTGGATTGATGTGGTGTG TGAGTGCGGTCCC | SEQ ID NO: 32 |
| | CCCAACGAGCGGCGTACGGAATTAATT CGCTAGCTGGAGCTTGGATTGATGTGG TGTGTCAGTGCGGAGCCC | SEQ ID NO: 33 |
| L-Dopa | TTAACCTAATGGAATACAATTCTAACA GGAATAGGAGGGTAGCTCCAAGTACT AACGACCGTTCCGGTAGCCGGCTGGCT | SEQ ID NO: 34 |
| | CGAATTGCCGTTGCACCGCATGTCAAA TCATGCGGCGGTTACATGTCGACGGCT CGGAAAAATCGGACAGCGC | SEQ ID NO: 35 |
| Kanamycin | ACCGCGGGTTGCGGACCGGGAGCTC CAGC | SEQ ID NO: 36 |
| | GAATTGCCGTAATTTCCCGTGGAGCGA TGC | SEQ ID NO: 37 |
| Streptomycin | UGUAACUCUAGGCUCCCGUGGAUCGC AUUUGGACUUCUGCCUAACGCGUGUG CUAGAGAGCAGGCAAACAAGUG | SEQ ID NO: 38 |
| | ACCCCUUUGUGGAACGCAAAACUAA AGGAAGAUCUCAAGAAAGGUGGAUC GCAUUUGGACUUCUGCCCAGGGU | SEQ ID NO: 39 |
| Neomycin | GGGAGAGGAUACUACACGUGAUACUC GUCCGUAGGAUGACCAGCACUAACUA GUCACAAGUCGGGUCCAUUGCAUGUA GCAGAAGCUUGGC | SEQ ID NO: 40 |
| Tobramycin | CUGGUUAGUUUUGCACAGUGGTCGAU GCUAGACUUGGUUUAGGUAAUGAGU CCAAUAGUC | SEQ ID NO: 41 |
| | AGUAUAGCGAGGUUUAGCUACACUCG UGCUGAUCGUUUGGUACGGGACCUGC GUGUAGCC | SEQ ID NO: 42 |
| Lividomycin | ACCGCGGGGTTGCGGACCGGGAGCTC CAGC | SEQ ID NO: 43 |
| | GAATTGCCGTAATTTCCCGTGGAGCGA TGC | SEQ ID NO: 44 |
| Moenomycin A | TGATGTCTCGAAATGAAGGAGGAGGA GGGGAACCGGATGG | SEQ ID NO: 45 |
| | AGATGTCTGAAGAACTCGGAGGAGGA GGCGGTCTGGCCTG | SEQ ID NO: 46 |
| Tetracycline | GCUUAAAGCCUAAAACAUACCAGAUC GCCACCCGCGCUUUAAUCUGGAGAGG UGAAGAAUUCGAC | SEQ ID NO: 47 |
| | UUAAAGCCUAAAACAUACCAGAUCGC UACCCGCGCUUUAAUCUGGGGAGGUG AAGAAUUCGAC | SEQ ID NO: 48 |

According to the invention, any redox label that is stable under assay conditions can be used. Examples of redox label include, but are not limited to, purely organic redox label, such as quinone derivatives, flavin derivatives (FAD, FMN), NAD, viologen, phenylene diamine derivatives, thianthrene derivatives, phtalocyanine derivatives, 2,2'-azino-bis(3-ethylbenzothiazoline)-6-sulfonic acid derivatives, 2,2-diphenyl-1-picrylhydrazyle derivatives, carbazol derivatives (Ar₂NH, diphenylamine, phenoxazine, phenothiazine), thiophene heterocyclic derivatives, nitroxyde derivatives, tetrathiafulvalene derivatives, methoxybenzene derivatives, anthraquinone, ethidium bromide, daunomycin, methylen blue and their derivatives; organo-metallic redox label, such as ferrocene, ruthenium, osmium, lanthanide, cobaltocenium, metalloporphyrin, metallophtalocyanin, cobalamin, bis-pyridine, tris-pyridine, bis-imidazole, and their derivatives; and biological label such as cytochrome c, plastocyanin, and cytochrome c'. The redox label can be attached to the target or target analog by any means, provided that it does not prevent the binding of the target or target analog to the oligonucleotide ligand. Examples of functional groups that can be used to attach the redox label to the target or target analog include, but are not limited to, amide, amine, sulfonamide, carbamate, thiocarbamate, aromatic amine, oxime, hydrazone, imine, ester, ether, carbonate, xanthate, triazole, triazole-cyclo-octyl, cyclohexene, thioester, azo derivatives, bis-thioether, thioether, alcene, biaryl, and alkyne.

Typically, the sample for electrochemical analysis is in liquid form, and preferably the sample is an aqueous mixture.

In one embodiment of the invention, the sample is a biological sample. Examples of biological samples include, but are not limited to, bodily fluids, preferably blood, more preferably blood serum, plasma, tears, saliva, sweat, synovial fluid, bronchoalveolar lavage fluid, sputum, lymph, ascitic fluids, urine, amniotic fluid, peritoneal fluid, cerebrospinal fluid, pleural fluid, pericardial fluid, and alveolar macrophages, tissue lysates and extracts prepared from diseased tissues.

In another embodiment, the sample may be derived from environmental sources.

In another embodiment, the sample contains a chemical compound. In one embodiment, said sample may comprise a chiral compound. In another embodiment, said sample may be derived from the crude a chemical synthesis.

According to the invention, the electrodes or electrode structure used for electrochemical detection can be fabricated from any known conductive and semi-conductive materials, such as for example gold, silver, platinum, carbon or silicon. The conductive and semi-conductive materials can be metallic or non-metallic. In one embodiment, the electrode surface may be treated by materials such as 2-mercaptoethanol, 6-mercaptohexanol, mercaptoalkanols in general (HS-(CH₂)ₙ-OH with n=2-18) or all mercapto derivatives which are soluble in aqueous solution, serum bovine albumin or the like. The electrode structure used in the invention comprises a working electrode from which the electrochemical readings will be taken, a reference electrode and optionally an auxiliary (counter) electrode , the electrodes being used in conjunction with a potentiostat and a sensitive current meter. A variety of electrochemical methods exploiting any two of the three parameters potential (E), current (i) and time (t) may be used to measure the electrochemical characteristics of the components. For example, electrochemical measurements can be made using differential pulse voltammetry, cyclic voltammetry or square-ware voltammery.

As used herein, the term "electrode" refers to any electrode of any size or shape and includes but is not limited to electrode, microelectrode or nanoelectrode.

In one embodiment of the invention, the electrodes or electrode structure used in the invention are treated by materials such as 2-mercaptoethanol, 6-mercaptohexanol, mercaptoalkanols in general (HS-(CH₂)ₙ-OH with n=2-18) or all mercapto derivatives wich are soluble in aqueous solution, bovine serum albumin, non ionic detergents at low concentration (Tween, Triton), polyvinylpyrrolidone, betaine, DNA samples, ß-globulin, poly-lysine, poly-ethylene-glycol, sugar or mixtures thereof. Preferably, the electrodes or electrode structure used in the invention are treated with bovine serum albumin.

According to the invention, the electrodes or electrode structure used for electrochemical detection can be used in combination or are comprised within a detector.

The term "detector" as used herein refers to a device, an apparatus or a support comprising at least one cell, chamber, cuvette or any other sample receiving vessel, wherein the electrode structure can be contacted with the sample. In one embodiment, the electrode structure can be part of the detector. In another embodiment, the electrode structure can form part of a probe for dipping into the sample.

Another object of the invention is a method for assessing and/or quantifying the presence of a target in a sample comprising the steps of
- contacting predetermined amounts of the oligonucleotide ligand with predetermined amounts of the redox labeled target or redox-labeled target analog in a detector comprising an electrode structure,
- detecting electrochemically a first redox signal, wherein the first redox signal is indicative of the complexes formed by the oligonucleotide ligand and the redox labeled target or redox-labeled target analog,
- contacting the sample with the detector of the invention,
- detecting electrochemically a second redox signal, wherein the second redox signal is indicative of the disruption of the previously formed complexes and thus of the presence of the target in the sample.

According to the invention, predetermined amounts of the oligonucleotide ligand and predetermined amounts of the redox labeled target or redox-labeled target analog, in a liquid form, are mixed in the detector to form a first complex and contacted to the electrode structure, thereby obtaining a first redox signal corresponding to said first complex. Then, the sample to be tested is added and in the presence of the target, the first complex is disrupted due to competition between the target and the redox label target or redox labeled target analog, and a second redox signal is detected.

The comparison between the first and the second redox signals allows the person skilled in the art to identify the presence of the target in the sample and to quantify it by reference to reference data furnished with the assay. Said reference data may be in the form of a calibration curve.

In another embodiment, the method of the invention can further comprise the steps of running the assay of the invention with at least two standard samples that contain known amounts of the target in serial dilution and optionally a negative control sample that comprises no target, in order to obtain a calibration curve. Said calibration curve may be used to determine the exact quantity of target in the sample to be tested.

The complex formation and disruption events that are detected by the detector of the invention are carried out in liquid environments. Any of a wide variety of buffers, including conventional buffers, may be employed such as MES, borate, SDS, PBS, borate/phosphate, phosphate, and the like may be employed, as well as buffers such as HEPES, MOPS, MES, Tricine, Tris, acetate, citrate and the like. The buffers may include additives such as alcohols, surfactants, detergents and the like.

Complex formation and disruption can be carried out in the presence of agents and additives that promote the desired binding, diminish non-specific background interactions or increase the stability of the oligonucleotide ligand. For example, one or more polyols may be added to the reaction. Representative polyols include glycerol, ethylene glycol propylene glycol sugars such as sucrose or glucose, and the like. One can also add similar levels of water soluble or water dispersible polymers such as polyethylene glycol (PEG) or polyvinyl alcohol or the like. Another representative additive is up to about 1 or 2% by weight (again based on the liquid substrate) of one or more surfactants such as triton X-100 or sodium dodecyl sulfate (SDS). All of these agents are electrochemically silent at the potentials observed with the detectors and methods of the invention.

In one embodiment, the method of the invention is carried out in duplicate, triplicate, or more using a detector as defined here above. Preferably, the method of the invention is carried out in a microwells or nanowells plates or arrays.

Another object of the present invention is a kit of parts comprising:
- a detector comprising an electrode structure for carrying out the assay of the invention,
- at least one container comprising at least one oligonucleotide ligand in a determined quantity or concentration, and
- at least one container comprising at least one redox labeled target or redox-labeled target analog in a determined quantity or concentration.

In one embodiment, the oligonucleotide ligand and the redox labeled target or redox-labeled target analog are already diluted in a buffer in the containers.

In another embodiment, the oligonucleotide ligand and the redox labeled target or redox-labeled target analog are lyophilized in the containers.

Optionally, said kit of parts comprises buffers for carrying out the assay.

Another object of the invention is a detector comprising an electrode structure for carrying out the assay of the invention, wherein the detector comprises at least one oligonucleotide ligand and at least one redox labeled target or redox-labeled target analog lyophilized in a determined quantity.

For example, the detector may be a microwells plate comprising microelectrodes, wherein the microwells comprise at least one oligonucleotide ligand and at least one redox labeled target or redox-labeled target analog lyophilized. Water or buffer is then used to dissolve the lyophilized reagents.

Another object of the invention is a kit of parts comprising:
- a detector comprising an electrode structure for carrying out the assay of the invention, wherein at least one oligonucleotide ligand in a determined quantity is attached to a solid phase or to an electrode, and
- at least one container comprising at least one redox labeled target or redox-labeled target analog in a determined quantity or concentration.

Another object of the invention is a multiplex method for assessing and/or quantifying the presence of multiple targets in a sample.

This method can be carried out by a detector comprising multiple electrode structures, wherein a predetermined amount of different oligonucleotide ligands is attached to each working electrode.

Multiple redox labeled target or redox-labeled target analog are provided for implementation of said multiplex method, wherein each redox labed used is different and has a redox potential differing by at least 50 millivolts, at least 100 millivolts, preferably at least 200 millivolts, from that of each other redox label.

For example, the detector comprises 2, 3, 4 or 5 working electrodes to which respectively 2, 3, 4 or 5 different oligonucleotide ligands are attached. 2, 3, 4 or 5 redox labeled targets or redox-labeled target analogs are provided for implementing the method, wherein said 2, 3, 4 or 5 redox labeled targets bind respectively to said 2, 3, 4 or 5 different oligonucleotide ligands and wherein 2, 3, 4 or 5 different redox labels are used, in order to detect 2, 3, 4 or 5 different redox signals.

Said multiplex method of the invention therefore comprises the steps of:
- contacting the multiplex detector as defined here above with predetermined amount of the multiple redox labeled targets or redox-labeled target analogs,
- detecting multiple first redox signals, wherein the first redox signals are indicative of the complexes formed by the multiple oligonucleotide ligands and the multiple redox labeled targets or redox-labeled target analogs,
- contacting the sample with the detector of the invention,
- detecting multiple second redox signals, wherein the second redox signals are indicative of the disruption of the previously formed complexes and thus of the presence of the multiple targets in the sample.

Another object of the invention is a kit of parts for implementing said multiplex method comprising:
- a detector comprising multiple electrode structures, wherein a predetermined amount of multiple oligonucleotide ligands is attached to each multiple working electrode, and
- multiple containers comprising one redox labeled target or redox-labeled target analog, wherein each target or target analog is different and each redox label used has a redox potential differing by at least 50 millivolts, at least 100 millivolts, preferably at least 200 millivolts.

Another object of the invention is a kit of parts for implementing said multiplex method comprising:
- a detector comprising nₑ electrode structures, wherein a predetermined amount of nₗ oligonucleotide ligands is attached to each nₑ working electrode, and
- nₜ containers comprising one redox labeled target or redox-labeled target analog, wherein each of nₜ target or target analog is different and each redox label used has a redox potential differing by at least 50 millivolts, at least 100 millivolts, preferably at least 200 millivolts,
wherein nₑ= nₗ= nₜ and is more than 1 and preferably 2, 3, 4 or 5.

One object of the present invention is the use of the assay, method and products of the invention for identifying and/or quantifying enantiomers or for determining enantiomeric excess. This can be implemented by the use of oligonucleotide ligands, preferably aptamers, that bind to each enantiomeric form of the molecular target.

This is particularly useful in food industry for assessing food quality through the enantiomeric analysis of amino acids, or in forensic science for discriminating and quantifying illicit chiral substances, or in environment for monitoring the fate of emerging chiral pollutants or in pharmaceutical field for enantiomeric analysis of biologically active molecules

The assay, method and products of the invention present the following advantages: simplicity, rapidity (less than 1 mn), low cost, and low volume sample (few µl).

Another object of the invention is an assay for assessing and/or quantifying the presence of at least one enantiomer in a sample, comprising:
- at least one oligonucleotide ligand capable of specifically interacting with the enantiomer of interest,
wherein the at least one enantiomer is naturally electrochemically active.

As used herein, the term "naturally electrochemically active" refers to a molecule that is capable to be electrochemically detected without any redox label being attached to the molecule.

Examples of naturally electrochemically active molecules include, but are not limited to, theophylline, malachite green, sulforhodamine B, hematoporphyrin, 4,40-methylenedianiline, dopamine, (R)-thalidomide, 17β-estradiol, reactive green 19, cibacron blue 3GA, reactive yellow 86, reactive brown 10, reactive blue 4, fluorescein, Hoechst dye 33258, malachite green, ATP, adenosine, cAMP, cyanocobalamin, riboflavin, FMN, FAD, NAD, hemin, tryptophan (such as, for example, D-tryptophan or L-tryptophan), tyrosinamide (such as, for example, D-tyrosinamide or L-tyrosinamide), L-dopa, tetracycline, chloramphenicol, oxytetracycline, ochratoxin A, and bisphenol A.

In one embodiment, the method for assessing and/or quantifying the presence of at least one enantiomer in a sample comprises the steps of
- contacting the sample with a detector comprising an electrode structure and detecting electrochemically a first redox signal, wherein the first redox signal is indicative of the presence of at least one naturally electrochemically active enantiomer in the sample,
- adding at least one oligonucleotide ligand that is specific for one enantiomer form (which means that said oligonucleotide ligand is not specific for the other enantiomer form),
- detecting electrochemically a second redox signal, wherein the second redox signal is indicative :
   i. of the presence of the other enantiomer to which the oligonucleotide ligand is not specific, if no decrease of the current is observed; and
   ii. of the presence of the enantiomer to which the oligonucleotide ligand is specific, if a decrease of the current is observed.

In another embodiment, another oligonucleotide ligand that is specific for the other enantiomer form may be further added and a third redox signal may be detected, to determine the presence of said other enantiomer form in the sample.

The enantiomeric excess may therefore be calculated from the second redox signal and/or from the third redox signal.

Another method for assessing and/or quantifying the presence of at least one enantiomer in a sample comprises the steps of
- contacting the sample with a detector comprising an electrode structure and detecting electrochemically a first redox signal, wherein the first redox signal is indicative of the presence of at least one naturally electrochemically active enantiomer in the sample,
- contacting the sample with a second detector comprising an electrode structure to which is attached at least one oligonucleotide ligand that is specific for one enantiomer form and detecting electrochemically a second redox signal, wherein the second redox signal is indicative of the presence of the enantiomer to which the oligonucleotide ligand is specific,
- contacting the sample with another detector comprising an electrode structure to which is attached at least one oligonucleotide ligand that is specific for the other enantiomer form and detecting electrochemically a third redox signal, wherein the third redox signal is indicative of the presence of the other enantiomer form to which the oligonucleotide ligand is specific.

Said method may be carried out for example in microwells plates, at least one well being dedicated to the measure of the first redox signal, at least another well being dedicated to the measure of the second redox signal and at least another well being dedicated to the measure of the third redox signal.

### BRIEF DESCRIPTION OF THE DRAWINGS

**Figure 1**: Electrochemically competitive assay of the invention. Left-hand column: before the addition of the sample: a first redox signal is measured. Right-hand column: after the addition of a sample containing the target to be detected or measured: a second redox signal is measured. (**A**) Homogeneous electrochemically competitive assay: the presence of the target disrupts the complex formed between the oligonucleotide ligand and the redox-labeled target. Thus, the second redox signal is superior to the first redox signal. (**B**) Heterologous electrochemically competitive assay, wherein the oligonucleotide ligand is attached to a solid phase: the presence of the target disrupts the complex formed between the oligonucleotide ligand and the redox-labeled target, thereby inducing a better detection of the redox labeled-target. Thus, the second redox signal is superior to the first redox signal. (**C**) Heterologous electrochemically competitive assay, wherein the oligonucleotide ligand is attached to the electrode: the presence of the target disrupts the complex formed between the oligonucleotide ligand and the redox-labeled target, thereby reducing the detection of the redox labeled-target. Thus, the second redox signal is inferior to the first redox signal.

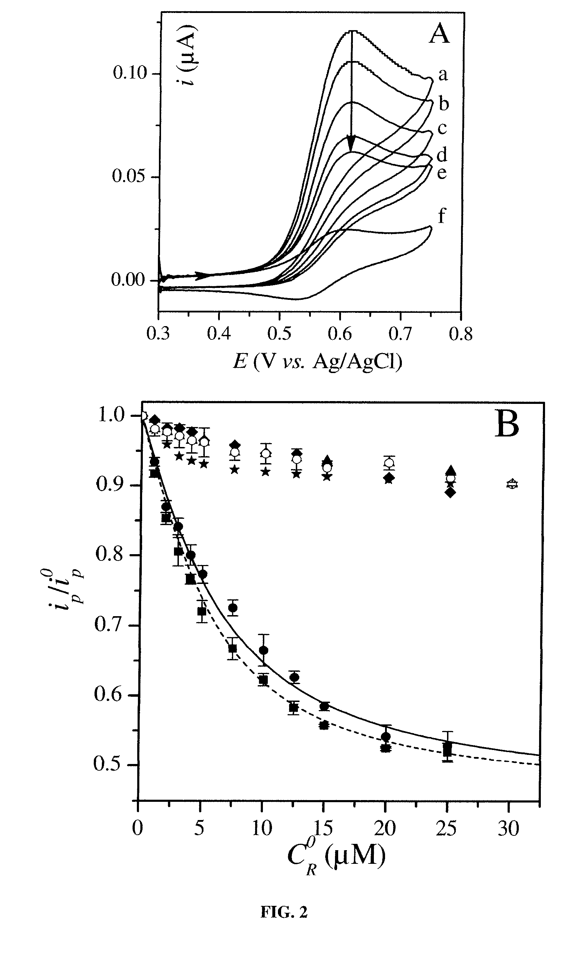

**Figure 2**: (A) CVs (v = 0.05 V s⁻¹) of 5 mM Tris buffer (pH 7.0) containing 50 mM NaCl, 10 mM MgCl₂, 1 µM [Os(bpy)₃]²⁺, 5 µM L-Tym and (a) 0 µM, (b) 2 µM, (c) 5 µM, (d) 10 µM (e) 20 µM Apt₄₉. (f) CV of 1 µM [Os(bpy)₃]²⁺ alone. (B) Normalized catalytic peak current as a function of aptamer concentration for the ligand/receptor couples: (●) L-Tym/D-Apt₄₉, (■) D-Tym/L-Apt₄₉, (○) D-Tym/D-Apt₄₉, (★) **3**/D-Apt₄₉, (◆) **4**/D-Apt₄₉, and (▲) L-Tym/49-mer scramble oligonucleotide. Error bars: standard deviations from 3 measurements. Plain lines: fits of eq 5 to the experimental data.

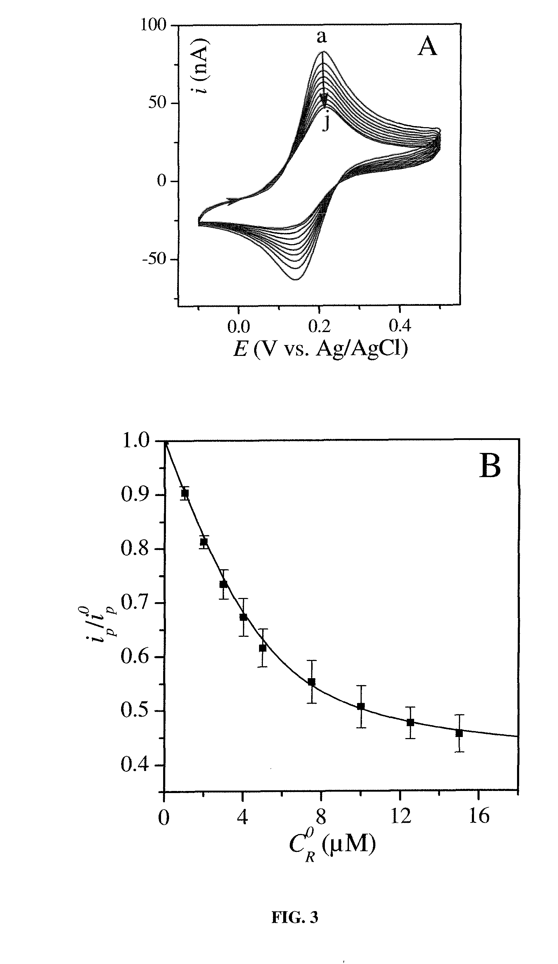

**Figure 3**: (A) CVs (v = 0.05 V s⁻¹) of 5 mM Tris buffer (pH 7.0) containing 50 mM NaCl, 10 mM Mg²⁺, 5 µM Fc-L-Tym and (a) 0 µM, (b) 1 µM, (c) 2 µM, (d) 3 µM, (e) 4 µM (f) 5 µM (g) 7.5 µM (h) 10 µM (i) 12.5 µM and (j) 15 µM of D-Apt₄₉. (B) Normalized anodic peak current response as a function of aptamer concentration. Each data is the average of 3 measurements. Error bars are standard deviations. Plain line: fit of eq 5 to the experimental data.

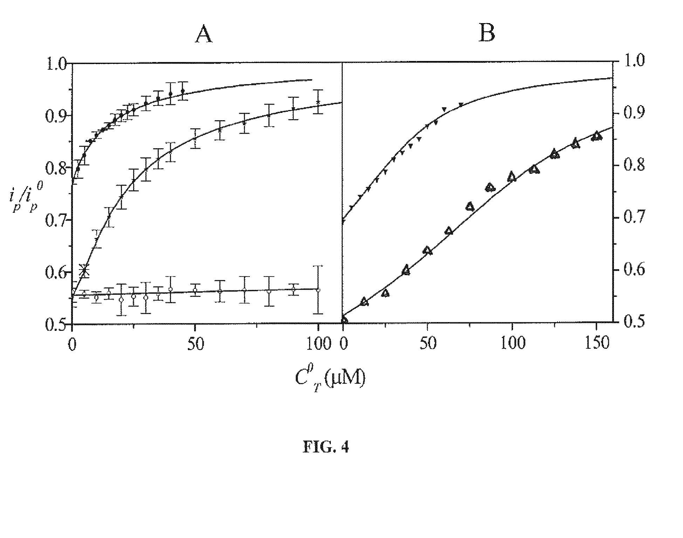

**Figure 4**: Competitive binding curves performed in (A) 5 mM Tris buffer (pH 7 + 50 mM NaCl + 10 mM MgCl₂) or (B) 20% fetal bovine serum (simply diluted with a 4 mM MgCl₂ aqueous solution) as a function of (●, ★, ▼, Δ) L-Tym or (○) D-Tym concentration. (*) Assay of 5 µM L-Tym in the presence of 5 mM D-Tym. The Fc-L-Tym concentration was always *C⁰*_{*T**}= 5 µM, whereas the D-Apt₄₉ concentrations were *C⁰_{R}*: (●) 3, (★,○,*) 10, (▼) 40 and (Δ) 100 µM. The plain curves represent the best fits of eq S 13 to the experimental data using: (●) *K*^{*}*_{d}* = 1.2 µM, *K_{d}* = 2.8 µM and ${i}_{p}^{∞} / {i}_{p}^{0} =$ $0.44 ;$ (★) *K*^{*}*_{d}* = 1.2 µM, *K_{d}* = 2.2 µM and ${i}_{p}^{∞} / {i}_{p}^{0} = 0.44 ;$ (▼) *K*^{*}*_{d}* = 75 µM, *K_{d}* = 9 µM and ${i}_{p}^{∞} / {i}_{p}^{0} = 0.15 ;$ (Δ) *K*^{*}*_{d}* = 75 µM, *K_{d}* = 11 µM and ${i}_{p}^{∞} / {i}_{p}^{0} = 0.15.$ Error bars: standard deviations from triplicates.

### EXAMPLES

The present invention is further illustrated by the following examples.

### 1. Chemicals and reagents

2-Amino-1,3-propanediol hydrochloride (Aldrich), 2-amino-1,3-propanediol (Aldrich), NaCl (Fluka), MgCl2 (Sigma), 1-tyrosinamide (Aldrich), d-tyrosinamide (Millegen, France), paracetamol (Aldrich) and tyrosine (Sigma), triphenylphosphine (Aldrich), diethylazidocarboxylate solution (DEAD) (Aldrich), and ferrocene methanol (Aldrich) were used without further purification. All syntheses were performed with freshly distilled solvents (THF and MeOH). The oligonucleotides (1-DNA and d-DNA) were synthesized and HPLC-purified by Eurogentec (Anger, France) and their concentration was controlled by UV-Vis spectroscopy using a Nanodrop 2000 spectrophotometer (ThermoFisher). The oligonucleotides sequences were:
Apt49-T: (SEQ ID NO: 1)
   5'-AATTCGCTAGCTGGAGCTTGGATTGATGTGGTGTGTGAGTGCGGTGCCC ― 3'
Scramble: (SEQ ID NO: 2)
   5'- CTATGACCCTACCTGCTGATGCGTAGCGAGACCCGGAATCTAACCCCCT ― 3'

All experiments were performed in 5 mM Tris buffer (pH 7) containing 10 mM MgCl2 and 50 mM NaCl (Recognition Buffer, RBr).
The L-Tym concentration is always 5 µM. For cyclic voltammetric experiments, 1 µM [Os(bpy)3]2+ with 5 µM 1-Tym in a droplet of 50 µL RBr was used. So, before each experiment, the droplet is shaking with a micropipette. This procedure is repeated until we obtain a reproducible and stable voltammogram.
Mother solution of Fc-1-Tym was a 12.5 M solution in DMF. This product is conserved at 4°C and seal with parafilm. Before each experiment, this solution is diluted 10 times in stabilized DMF, such as 0.2 µL in 50 µL of RBr leads to 5 µM of Fc-1-Tym.

### 2. Instrumentation

All NMR spectra were recorded on a Brucker AC 400 MHz. Optical rotations were measured using a Jasco-P-1010 polarimeter.

Cyclic voltammetry measurements were performed with an AUTOLAB PGSTAT 12 potentiostat/galvanostat controlled by computer, and the data were acquired using GPES 4.9007 software (EcoChemie, The Netherlands). Small self-contained electrochemical cells made of 3 screen-printed electrodes on a flat substrate were used for all experiments. The electrodes were printed using a DEK model-65 screen-printer. Working electrodes were 4 mm diameter screen-printed carbon electrodes prepared using a commercial carbon ink (Electrodag PF-470 A) deposited on the top of an underlying Ag layer (Electrodag 418 SS). Counter electrode was obtained using an Ag ink (Electrodag 418 SS).

Before experiments, the screen-printed carbon electrodes were coated with bovine serum albumin (BSA) by immersing the electrodes in a 1 mg/mL BSA solution for 10 minutes at room temperature. The BSA-modified electrodes were next cycled between 0 V and 0.8 V at v = 0.05 V/s, until of the capacitive background current in cyclic voltammetry was stablized.

### 3. Syntheses

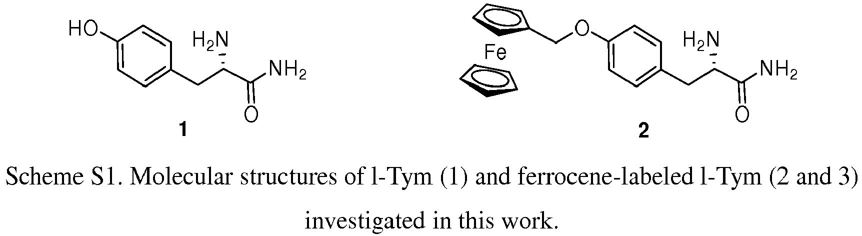

### O-Ferrocenyltyrosynamide (2) (Fc-Tym)

L-tyrosinamide 1 (250 mg, 1.39 mmol,) and triphenylphosphine (361 mg, 1 eq.) were added under argon at room temperature to a solution of ferrocenemethanol (200 mg, 0.7 eq.) previously dissolved in 5 mL of dry THF. After cooling the solution to ―30 °C, a 2 M solution of DEAD in THF (0.5 mL, 1 eq.) was slowly added. 20 mL of dry THF followed by 0.5 mL of dry methanol were then added to increase the solubility. The solution was stirred at room temperature for two days. The mixture was evaporated and submitted to a column chromatography using a mixture of CH₂Cl₂ with 1 to 10% MeOH. Compound 2 was obtained as a pale yellow solid in 67% yield. 1H NMR, MeOD, 400 MHz : δ (ppm)= 2.84 (dd, 1H, J_{AB} = 13.6 Hz, J_{AX} = 7.5 Hz, CH₂), 3.05 (dd, 1H, J_{AB} = 13.6 Hz, J_{AX} = 5.8 Hz, CH₂), 3.60-3.63 (m, 1H, CH), 4.27-4.28 (m, 7H, CHAr-Fc), 4.390 (t, 2H, J = 1.8 Hz, CHAr-Fc), 4.90 (s, 2H, CH₂O), 7.00 (d, 2H, J = 8.6 Hz, CHAr-Cq-O), 7.24 (d, 2H, J = 8.6 Hz, CHAr-Cq-CH₂). 13C NMR, MeOD, 100 MHz : δ (ppm) = 41.6(CH₂Ph), 57.4 (CH-NH₂), 67.9 (CH₂-O), 69.5 (CH-Fc), 69.6 (CH-Fc), 70.3 (CH-Fc), 84.1 (Cq-Fc), 116.0 (CHAr), 130.8 (CqAr), 131.4 (CHAr-O), 159.3 (CqAr-O), 179.5 (Cq-C(O)).
[α]D 31 = +22.1 (c = 0.21, MeOH)

### Results

The methodology is here exemplified with the enantioselective molecular recognition of trace amounts of L- or D-tyrosinamide (Scheme 1) by a 49-mer D- or L-deoxyribooligonucleotide receptor.

To demonstrate the feasibility of the method, we have first taken advantage of the intrinsic electroactivity of tyrosinamide and therefore of its electrochemical detection by cyclic voltammetric (i.e., oxidation of the phenol function) in the presence of its 49-mer anti-L-tyrosinamide aptamer (D-Apt₄₉). The 49-mer aptamer we have used (see here above) is a sequence similar to the one that was previously selected by Vianini et al and successfully applied to chromatographic- and fluorescence-based L-Tym assays. Since direct electrochemical oxidation of phenolic compounds generally leads to electrode passivation and thus to poorly reproducible anodic peak current in cyclic voltammetry (CV), the electrochemical detection of L-Tym was performed by redox-mediated catalytic oxidation of the phenolic function. For such a purpose, we have used the one-electron redox mediator couple [Os^{III/II}(bpy)₃]^{3+/2+} which, according to its relatively high standard potential (*E⁰*=+0.58 V vs. Ag/AgCl), is capable to efficiently catalyze the oxidation of phenolic compounds.

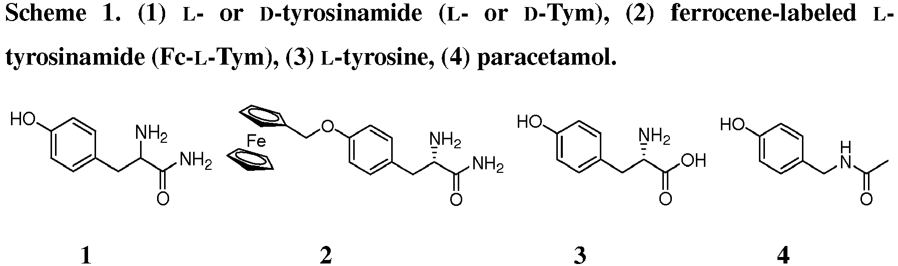

As illustrated in Figure 2, for particular scan rates and concentrations of L-Tym and [Os(bpy)³]²⁺, we obtained in CV a well-defined irreversible anodic catalytic peak current at +0.62 V vs. Ag/AgCl (Figure 2A). The linear dependence of the peak current with both the square root of scan rate, *v*, and L-Tym concentration, indicates conditions of total catalysis for which the catalytic peak current response, *iₚ*, can be expressed by eq 1. ${i}_{p} = 0.609 ⁢ nFS ⁢ {C}_{T}^{0} ⁢ \sqrt{{D}_{T}} ⁢ \sqrt{fv / RT}$ where *n* is the number of electron involved in the catalytic reaction, *F* the Faraday constant, *S* the electrode area, *C⁰_{T}* the bulk concentration of L-Tym, and *D_{T}* the diffusion coefficient of L-Tym. After aptamer addition, the binding reaction (eq 2) is rapidly set at equilibrium. 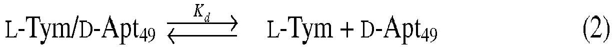 with *K*_{d} the dissociation constant. On the basis of a classical law of action mass and mass balance (i.e., *C⁰_{T}* = *C_{T}* + *C_{RT},* with *C_{T}* and *C_{RT}* the free and receptor-bound target concentrations), and assuming a 1:1 binding stoichiometry, the free (*χ_{ƒ}*) mole fraction of target in the bulk solution can thus be estimated by eq 3. ${χ}_{f} = \frac{{C}_{T}}{{C}_{T}^{0}} = 1 - \frac{{K}_{d} + {C}_{T}^{0} + {C}_{R}^{0} - \sqrt{{\left({K}_{d}+{C}_{T}^{0}+{C}_{R}^{0}\right)}^{2} - 4 ⁢ {C}_{T}^{0} ⁢ {C}_{R}^{0}}}{2 ⁢ {C}_{T}^{0}}$

Here, *C⁰_{R}* is the total concentration of the aptamer receptor added to the solution. Under the assumption of a slow interconversion of free and bound target on the time scale of CV experiments, the catalytic peak current can thus be expressed by a linear combination of the catalytic currents related to the free and bound target through eq 4. ${i}_{p} = 0.609 ⁢ nFS ⁢ {C}_{T}^{0} ⁢ \sqrt{fv / RT} ⁢ \left[{χ}_{f}⁢\sqrt{{D}_{T}}+\left(1-{χ}_{f}\right)⁢\sqrt{{D}_{RT}}\right]$
where *D_{T}* and *D_{RT}* are the diffusion coefficients of the free and receptor-bound target, respectively. Because of its greater gyration radius, the deoxyribooligonucleotide receptor/L-Tym complex is expected to diffuse significantly slower that the free L-Tym target (i.e., *D_{RT}*<<*D_{T}*). Consequently, according to eq 5, a decrease of the total catalytic current would result from increasing addition of D-Apta₄₉ in solution.

From the combination of eqs 3 and 5 and knowledge of the catalytic peak currents determined both in the absence of aptamer $\left({i}_{p}^{0}\right)$ and at extrapolated infinite aptamer concentration $\left({i}_{p}^{∞}\right) ,$ we can write eq 5 which allows to predict the normalized peak current response decreases as a function of aptamer and target concentrations. $\frac{{i}_{p}}{{i}_{p}^{0}} = 1 - \frac{{K}_{d} + {C}_{T}^{0} + {C}_{R}^{0} - \sqrt{{\left({K}_{d}+{C}_{T}^{0}+{C}_{R}^{0}\right)}^{2} - 4 ⁢ {C}_{T}^{0} ⁢ {C}_{R}^{0}}}{2 ⁢ {C}_{T}^{0}} ⁢ \left(1-\frac{{i}_{p}^{∞}}{{i}_{p}^{0}}\right)$

**Figure 2** shows the resulting asymptotic catalytic peak current response decrease as a function of D-Apt₄₉ concentration added to a fixed concentration of L-Tym. The nonlinear regression fit of eq 5 to the experimental plot (**Figure 2B**) allows for extracting the values of *K_{d}* = 2.9 ± 0.3 µM and ${i}_{p}^{∞} / {i}_{p}^{0} = 0.45.$ The value of *K_{d}* is in excellent agreement with the ones previously determined by isothermal titration calorimetry (1.75-3.2 µM) and fluorescence polarization (1.7-2.2 µM). From the ratio ${i}_{p}^{∞} / {i}_{p}^{0}$ and the experimental ${i}_{p}^{0}$ value, we can determine ${i}_{p}^{∞}$ as well as the diffusion coefficients of the free and bound L-Tym. Assuming one electron exchanged in the reaction, values of *D_{T}* = 7 × 10⁻⁶ cm² s⁻¹ and *D_{RT}* = 1.4 × 10⁻⁶ cm² s⁻¹ were found, respectively, which is in good agreement with the expected diffusion coefficient of an amino-acid and a small oligonucleotide in water at room temperature.

To examine the specificity as well as the enantioselectivity of the assay, binding experiments with the D-Tym enantiomer and two structurally related ligands (i.e., the L-tyrosine and paracetamol, both containing an oxidizable phenol group, Scheme 1) were performed. As reported in **Figure 2B**, no significant catalytic current response decrease was observed upon the addition of D-Apta₄₉ to a solution of D-Tym, indicating that the cross-reactivity of the D-aptamer receptor with the D-Tym enantiomer is negligible. Similar results were also obtained with the noncognate L-tyrosine and paracetamol ligands (**Figure 2B**), demonstrating the strong discriminating properties of D-Apta₄₉ against closely related compounds and the relevance of the proposed methodology. Finally, no significant current response decrease could be observed when the aptamer was replaced by a 49-mer random DNA sequence containing the same GC ratio.

We have next examined the possibility to perform the assay of D-Tym with the mirror image of D-aptamer, i.e. the L-Apt₄₉. Similarly to the titration curve of L-Tym by D-Apt₄₉, the catalytic peak current response of D-Tym was observed to rapidly decrease with the increasing addition of L-Apt₄₉ (**Figure 2B**), providing an equal binding curve to that of L-Tym. From the nonlinear regression fit of eq 5, a *K_{d}* value of 3.8 ± 0.7 µM was obtained, which is practically the same to the one determined for the mirror assay (i.e., 2.9 ± 0.3 µM). From the combination of the two specific responses of D- and L-aptamer toward their respective L- and D-target, it becomes conceivable to determine trace amounts of D- or L-Tym in the presence of a large excess of the L- or D-enantiomer. However, with the present redox-mediated electrochemical detection strategy, such an idea cannot be easily achieved and it is also restricted to target analytes that are intrinsically electroactives. We have thus extended the methodology to a more general detection scheme based on an electrochemical homogeneous competitive binding assay. In this approach, the target is reacted in competition with a known amount of the redox-labeled target for binding to the aptamer, and the ratio of free to bound redox-labeled target is monitored electrochemically.

The competitive assay can be described by the two equilibrated binding reactions 6a and 6b, in which Fc-L-Tym is L-tyrosinamide labeled by a redox-active ferrocene group. The ferrocenyl label was here incorporated on the ―OH position of the phenolic group (Scheme 1). Note that under this condition the catalytic oxidation of the phenolic residue is no more possible and only the ferrocene labeled molecule can be detected. 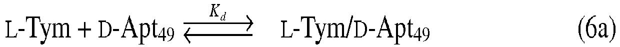 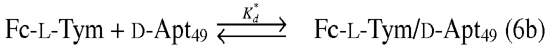 with ${K}_{d} = \left({C}_{T}⁢{C}_{R}\right) / {C}_{RT}$ ${K}_{d}^{*} = \left({C}_{{T}^{*}}⁢{C}_{R}\right) / {C}_{{RT}^{*}}$
where *C_{R}* is the concentration of free aptamer receptor. For successful achievement of such an assay, it is important that the labeling does not significantly affect the aptamer molecular recognition. Ideally, the two binding constants should be as close as possible (*K_{d}* ≈ *K*^{*}*_{d}*). The value of *K*^{*}*_{d}* was determined by monitoring in CV the reversible response of 5 µM Fc-L-Tym solution as a function of increasing concentrations of D-Apt₄₉. The simultaneous decrease of the anodic and cathodic peak currents upon the addition of D-Apt₄₉ sequence (**Figure 3A**) and the absence of current change in the presence of L-Apt₄₉ (not shown), clearly shows that the labeled target is well recognized by the aptamer and that the ferrocene label is less easily electrochemically detected when Fc-L-Tym is bound to D-Apt₄₉ in solution.

Although the expression of the peak current is here different from the one previously used for the catalytic response (it is given by ${i}_{p} = 0.4463 ⁢ {FSC}_{Fc}^{0} ⁢ \sqrt{{D}_{Fc}} ⁢ \sqrt{Fv / RT} ) ,$ the same eq 5 can be applied to fit the experimental plot of the anodic peak current decrease of ferrocene as a function of aptamer concentration (**Figure 3B**). From the data fitting, a value of *K*^{*}*_{d}* = 1.2 ± 0.4 µM was determined, which is ~2.5-fold lower than the dissociation constant obtained for L-Tym. This slightly stronger affinity binding clearly shows that the labeling of L-Tym through the activable hydroxyl group does not hinder the aptamer binding (it is rather the opposite here), and so that the free ―OH function does not significantly contribute to the molecular recognition of L-Tym. This is not the case of the primary ―NH₂ function because when the ferrocene moiety was covalently linked to this position (Scheme 1) via an amide or a secondary amino group, the aptamer was unable to bind these labeled L-tyrosinamides. From the fit in **Figure 3B**, a ${i}_{p}^{∞} / {i}_{p}^{0}$ value of 0.40 was also found, which is close to the one previously determined for L-Tym. This result was expected since the diffusion coefficients of free or bound Fc-L-Tym and free or bound L-Tym are nearly the same.

The competitive binding assay was achieved from two separate solutions containing a starting mixture of 5 µM Fc- L-Tym and 3 µM or 10 µM D-Apt₄₉, and to which an increasing amount of L-Tym was added. On account of the competitive displacement of the aptamer-bound Fc- L-Tym by L-Tym, peak currents of the reversible voltammetric wave of ferrocene were observed to progressively increase with the L-Tym concentration, allowing for plotting the typical competitive calibration curves shown in **Figure 4A**. Solving the cubic algebraic equation that results from combination of eqs 7a, 7b and equations of mass balance, an explicit analytical expression can be obtained and used to fit the experimental competitive binding calibration curves. From the best nonlinear regression fits and the predetermined value of *K*^{*}*_{d}* = 1.2 µM, the parameters *K_{d}* and ${i}_{p}^{∞} / {i}_{p}^{0}$ were extracted from the two competitive curves in **Figure 4A**. An identical ${i}_{p}^{∞} / {i}_{p}^{0}$ value of 0.44 was obtained, and similar *K_{d}* of 2.8 ± 0.4 µM and 2.2 ± 0.6 µM were found, which is in very good agreement with the dissociation constant of L-Tym determined from redox-mediated catalysis.

The enantiospecificity of the assay was next characterized using the same methodology but for solutions containing the D-Tym enantiomer. As shown in **Figure 4A**, no displacement of Fc- L-Tym from D-Apt₄₉ by D-Tym could be observed over the 0-100 µM concentration range. Even concentrations as high as few millimolar D-Tym were tested, but again without significant response change. These results once more demonstrate that the cross-reactivity with the enantiomer is negligible. To evaluate the enantiospecific performance of the aptamer, the assay of trace amount of L-Tym in the presence of a large excess of D-Tym was examined and, as reported in **Figure 4A**, it has been found possible to detect as low as 5 µM L-Tym in the presence of 5 mM D-Tym. This remarkable performance means that as low as 0.1 % of an enantiomeric impurity in a nonracemic mixture can be assayed in a very short analysis time (<1 min). This is competitive with conventional separation techniques, and one order of magnitude lower than the 1% currently attainable by NMR. Finally, the ability to perform an assay under realistic biological conditions was examined. For such purpose, homogenous electrochemical competitive binding assays of 5-fold diluted fetal bovin serum samples spiked with known amounts of Fc-LTym were then performed (**Figure 4B**). The competitive binding plots under these conditions were observed to be slightly shifted towards higher concentrations, indicating a lower sensitivity assay in diluted serum than in pure buffer. From the nonlinear fit of the experimental curves, average values of *K*^{*}*_{d}* = 75 µM and Kd = 10 µM were obtained, demonstrating a lower affinity binding of the aptamer towards both the L-Tym and the Fc-LTym in the presence of serum. Regardless this minor issue, the applicability of the method in complex biological samples is finally demonstrated.

## Claims

1. An electrochemical competitive assay for assessing and/or quantifying the presence of at least one target in a sample, comprising
- at least one oligonucleotide ligand capable of specifically interacting with the target of interest,
- at least one redox-labeled target or redox-labeled target analog,
wherein the oligonucleotide ligand and the redox-labeled target or redox-labeled target analog form a first complex allowing a first redox signal to be electrochemically detectable and
wherein, when the sample to be tested is added, the first complex is disrupted by the target present in the sample allowing a second redox signal to be electrochemically detectable,
said second redox signal allowing the assessment and/or quantification of the target presence in the sample.

2. The electrochemical competitive assay according to claim **1**, being an homogeneous assay.

3. The electrochemical competitive assay according to claim **1**, being an heterogeneous assay, wherein the oligonucleotide ligand is attached to a solid phase or to an electrode.

4. The electrochemical competitive assay according to any one of claims **1** to **4**, wherein said oligonucleotide ligand is an aptamer, a DNAzyme, a ribozyme, a PNA, or a LNA.

5. The electrochemical competitive assay according to any one of claims **1** to **4**, wherein said redox label is an organic redox label, an organo-metallic redox label or a biological label.

6. The electrochemical competitive assay according to any one of claims **1** to **5**, wherein said sample is a biological sample, a sample derived from an environmental source or a sample containing a chemical compound.

7. A method for assessing/quantifying the presence of at least one target in a sample comprising the steps of:
- contacting the oligonucleotide ligand with the redox labeled target or redox-labeled target analog in a detector comprising an electrode structure,
- detecting electrochemically a first redox signal, wherein the first redox signal is indicative of the complexes formed by the oligonucleotide ligand and the redox labeled target or redox-labeled target analog,
- contacting the sample with the detector,
- detecting electrochemically a second redox signal, wherein the second redox signal is indicative of the disruption of the previously formed complexes and thus of the presence of the target in the sample.

8. The method according to claim **7**, further comprising the step of running the assay with at least two standards samples, thereby obtaining a calibration curve.

9. The method according to any one of claims **7** to **8**, for multiplex detection and/or quantification of multiple molecular targets in a sample, wherein the redox label used are different and have a redox potential differing by at least 50 millivolts.

10. A kit of parts comprising:
- a detector comprising an electrode structure,
- at least one oligonucleotide ligand,
- at least one redox labeled target or redox labeled target analog.

11. The kit according to claim **10**, wherein the oligonucleotide ligand and the redox labeled target or redox labeled target analog are in two separate containers.

12. The kit according to claim **10**, wherein the oligonucleotide ligand and the redox labeled target or redox labeled target analog are lyophilized and contained in the detector.

13. The kit according to claim **10**, wherein the oligonucleotide ligand is attached to a solid phase or to an electrode and the redox labeled target or redox labeled target analog is in a separate container.

14. The kit according to claim **10** for implementing a multiplex method, wherein the detector comprises multiple electrode structures to which are attached multiple oligonucleotide ligands and wherein the multiple redox labeled targets or redox labeled target analogs are in separate containers, the multiple redox label used being different and having a redox potential differing by at least 50 millivolts.

15. The use of the assay, methods and kits according to anyone of claims **1** to **14** for assessing and/or quantifiying enantiomers or an enantiomeric excess in a sample.
